# EUROPÄISCHE PATENTSCHRIFT

(11) **EP 3 595 557 B1**
(45) Veröffentlichungstag und Bekanntmachung des Hinweises auf die Patenterteilung: **16.11.2022**
(21) Anmeldenummer: 18701299.2
(22) Anmeldetag: 17.01.2018
(51) Int. Cl.: A61B 17/80

(54) **TTA-IMPLANTATSYSTEM**
TTA IMPLANT SYSTEM
SYSTÈME D'IMPLANT TTA

(30) Priorität: 17.03.2017 DE 102017105768
(43) Veröffentlichungstag der Anmeldung: 22.01.2020
(73) Patentinhaber: Rita Leibinger GmbH & Co. KG, 78570 Mühlheim an der Donau (DE)
(72) Erfinder: LEIBINGER, Rita, 78570 Muehlheim (DE); BUSCHLE, Nelson, 78532 Tuttlingen (DE); SCHMOEKEL, Hugo, 73492 Kolbaeck (SE)
(74) Vertreter: DREISS Patentanwälte PartG mbB
(86) Internationale Anmeldenummer: PCT/EP2018/051050
(87) Internationale Veröffentlichungsnummer: WO 2018/166670

(56) Entgegenhaltungen:
- WO-A1-2013/179142
- US-A1- 2010 076 564
- US-A1- 2013 338 781
- US-A1- 2016 184 099

## Beschreibung

Die Erfindung betrifft ein TTA-Implantatsystem nach dem Oberbegriff des Anspruchs 1.

Ein TTA-Implantat ist aus der DE 10 2011 079 821 A1 bekannt. TTA ist die Abkürzung für "Tibial Tuberosity Advancement". Hierdurch wird eine Degeneration des cranialen Kreuzbands behandelt, durch die die Stabilität eines Knies beispielsweise eines Hundes oder einer Katze verringert wird. TTA-Implantate werden also vor allem in der Veterinärmedizin dazu eingesetzt, die Tibia Tuberosity nach vorn zu verlagern, wodurch der Winkel der Patellasehne verändert wird. Hierdurch wiederum wird die tibiafemurale Scherkraft bei Belastung neutralisiert. Hierzu wird die Tibia im knienahen Bereich an der Vorderseite von oben bereichsweise aufgespaltet und abgespreizt, so dass ein keilförmiger Einschnitt entsteht. In diesen Einschnitt wird das TTA-Implantat mit einem distalen Bereich voraus eingeschoben, so dass der aufgespaltene Bereich abgespreizt und die Tibia Tuberosity nach vorn verlagert bleibt. Das bekannte TTA-Implantat umfasst einen keilförmigen Implantatkörper, von dessen proximalem Bereich mehrere Befestigungslaschen seitlich abstehen. Mittels dieser Befestigungslaschen wird der Implantatkörper mithilfe von Knochenschrauben an der Tibia befestigt.

Weitere TTA-Implantate sind aus der WO 2013/179142 A1, der US 2013/0338781 A1 und der US 2010/0076564 A1 bekannt. In der Praxis hat sich gezeigt, dass es wünschenswert ist, die Rotationsstabilität der Gelenkverbindung (im allgemeinen also eines Knies), in deren Bereich das TTA-Implantat eingesetzt ist, zu verbessern.

Diese Aufgabe wird durch ein TTA-Implantatsystem mit den Merkmalen des Anspruchs 1 gelöst. Vorteilhafte Weiterbildungen der Erfindungen sind in Unteransprüchen angegeben. Darüber hinaus finden sich für die Erfindung wesentliche Merkmale in der nachfolgenden Beschreibung und in der beigefügten Zeichnung.

Das erfindungsgemäße TTA-Implantatsystem umfasst einen Implantatkörper zum Einsetzen in einen Knochenspalt und eine an einen proximalen Bereich des Implantatkörpers angeformte Befestigungseinrichtung zum Befestigen des Implantatkörpers am Knochen. Erfindungsgemäß wird vorgeschlagen, dass das TTA-Implantat ferner eine zweite Befestigungseinrichtung umfasst, die in einem distalen Bereich des Implantatkörpers angeordnet ist und mindestens einen an und/oder in dem Implantatkörper ausgebildeten Befestigungsmittelkanal umfasst, der so ausgebildet und dimensioniert ist, dass durch ihn eine flexibles Befestigungsmittel hindurchgeführt werden kann.

Durch die zweite Befestigungseinrichtung, die insbesondere als Befestigungsmittelkanal ausgebildet ist oder einen solchen umfasst, ist es möglich, dass TTA-Implantat zusätzlich mit dem auf der anderen Seite des Gelenks vorhandenen Knochen zu verbinden. Entsprechend umfasst das erfindungsgemäße TTA-Implantatsystem ein solches flexibles Befestigungsmittel, welches durch den Befestigungsmittelkanal des TTA-Implantats hindurchgeführt werden kann, sowie eine Verankerungseinrichtung, insbesondere eine Knochenschraube, die an einer vom TTA-Implantat entfernten Stelle im Knochen auf der anderen Seite des Gelenks (also an einem anderen Knochen) verankert werden kann und die so ausgebildet ist, dass an ihr das flexible Befestigungsmittel festgelegt werden kann. Vorzugsweise wird die besagte Verankerungseinrichtung vom implantierten TTA-Implantat ausgesehen nach schräg seitlich oben angeordnet, so dass mithilfe des flexiblen Befestigungsmittels ein Wegdrehen des anderen Knochens (beispielsweise Femur oder Tibia) relativ zu dem Knochen (beispielsweise Tibia oder Femur), an dem das TTA-Implantat befestigt ist, verhindert wird. Durch die erfindungsgemäß vorgesehene zweite Befestigungseinrichtung erfüllt das TTA-Implantat also eine Doppelfunktion: zum einen trägt es dazu bei, die Bänder im Bereich eines Gelenks zu straffen, und zum anderen wird ein einfacher aber sehr stabiler Verankerungspunkt für das flexible Befestigungsmittel geschaffen, welches für eine zusätzliche Stabilisierung der relativen Rotation der beiden durch das Gelenk verbundenen Knochen sorgt.

Bei einer Weiterbildung wird vorgeschlagen, dass es eine Mehrzahl von flexiblen Befestigungsmitteln umfasst, die durch den Befestigungsmittelkanal hindurchgeführt werden können. Damit wird eine besonders zuverlässige und stabile Stabilisierung der Rotation des Gelenks ermöglicht.

Das flexible Befestigungsmittel kann einen Nahtfaden, insbesondere aus einem Monofilamentmaterial oder aus einem Mulitfilamentmaterial, umfassen. Diese sind preiswert, beim Implantieren des TTA-Implantats ohnehin vorhanden und sehr stabil sowie einfach handhabbar.

In einer Weiterbildung wird vorgeschlagen, dass der Befestigungsmittelkanal in eine Stirnseite des Implantatkörpers mündet. Eine solche Stirnseite kann bei implantiertem TTA-Implantat zum Gelenk und somit zu dem anderen Knochen hin zeigen, was für die optimale Führung des flexiblen Verbindungsmittels hilfreich ist und was auf diese Weise auch den Implantiervorgang und das Befestigen des Befestigungsmittels am TTA-Implantat erleichtert.

Ebenso ist es möglich, dass der Befestigungsmittelkanal in mindestens eine laterale Seitenfläche, vorzugsweise in beide lateralen Seitenflächen des Implantatkörpers mündet. Hierdurch wird eine erhöhte Flexibilität beim Einsatz des TTA-Implantats geschaffen.

Ferner wird vorgeschlagen, dass der Implantatkörper mindestens eine Durchgangsöffnung aufweist, welche Teil des Befestigungsmittelkanals ist. Derartige Durchgangsöffnungen sind bei vielen TTA-Implantaten ohnehin vorhanden um einerseits Material zu sparen und um ein Durchwachsen des TTA-Implantats mit Knochensubstanz im Laufe der Zeit zu ermöglichen. Bei dieser Weiterbildung sind daher zur Herstellung der erfindungsgemäßen Zusatzfunktion nur geringe Änderungen an dem TTA-Implantat erforderlich.

Eine andere Weiterbildung zeichnet sich dadurch aus, dass der Implantatkörper eine Mehrzahl von Plattenabschnitten aufweist, die sich quer zu einer ersten Richtung des Implantatkörpers erstrecken, die sich vom proximalen zum distalen Ende hin erstreckt, und dass die zweite Befestigungseinrichtung, insbesondere der Befestigungsmittelkanal in oder an mindestens einem der Plattenabschnitte ausgebildet ist. Solche aus einer Mehrzahl von parallelen Platten, die meist durch einen zentralen Steg miteinander verbunden sind, aufgebaute Implantatkörper sind sehr preiswert. Darüber hinaus kann beispielsweise ein Befestigungsmittelkanal in einer solchen Platte einfach durch Einbringen einer Bohrung hergestellt werden, was ebenfalls sehr preiswert ist.

In einer konkreten Weiterbildung hierzu wird vorgeschlagen, dass zu beiden Seiten eines Plattenabschnitts jeweils ein Befestigungsmittelkanal vorhanden ist. Dies erhöht die Einsatzflexibilität des erfindungsgemäßen TTA-Implantats.

In die gleiche Richtung geht jene Weiterbildung der Erfindung, bei der die zweite Befestigungseinrichtung eine Mehrzahl von Befestigungsmittelkanälen umfasst.

Die Zuverlässigkeit des TTA-Implantats wird erhöht, wenn ein Mündungsrand des Befestigungsmittelkanals abgeschrägt oder abgerundet ist. Beschädigungen an dem flexiblen Befestigungsmittel im Laufe der Zeit werden hierdurch verhindert oder zumindest verringert.

Der Befestigungsmittelkanal kann mindestens bereichsweise einen kreisförmigen, elliptischen oder mehreckigen QuerschniTTAufweisen. Hierdurch wird für jede individuelle Einsatzsituation ein jeweils optimales TTA-Implantat geschaffen.

Für eine stabile Abspreizung eines Knochenabschnitts und eine langfristige Stabilisierung des abgespreizten Knochenabschnitts ist es hilfreich, wenn sich der Querschnitt des Implantatkörpers in einer zweiten Richtung, die sich quer zu einer ersten Richtung des Implantatkörpers erstreckt, die sich vom proximalen zum distalen Ende hin erstreckt, verjüngt.

Jene Weiterbildung, bei welcher ein Rand am distalen Ende gegenüber einem Rand am proximalen Ende schräg ist, bildet eine gelenksnahe Knochenkontur besonders gut ab, wodurch überstehende Bereiche des TTA-Implantats vermieden werden. Vorgeschlagen wird auch, dass der Implantatkörper aus Metall, Kunststoff, Kohlefaser, Polyetheretherketon, einem resorbierbaren Material, oder einer Kombination hiervon hergestellt ist. Damit können ganz unterschiedliche Einsatzszenarien abgedeckt werden.

Nachfolgend werden mögliche Ausführungsformen der Erfindung unter Bezugnahme auf die beigefügte Zeichnung erläutert. In der Zeichnung zeigen:
- Figur 1: eine Seitenansicht auf ein Kniegelenk mit einem TTA-Implantatsystem mit einem schematisch gezeigten TTA-Implantat;
- Figur 2: eine perspektivische Darstellung der Situation von Figur 1;
- Figur 3: eine erste perspektivische Ansicht einer ersten Ausführungsform des TTA-Implantats der Figuren1 und 2;
- Figur 4: eine zweite perspektivische Ansicht des TTA-Implantats von Figur 3;
- Figur 5: eine dritte perspektivische Ansicht des TTA-Implantats von Figur 3;
- Figur 6: eine Frontansicht des TTA-Implantats von Figur 3;
- Figur 7: eine Seitenansicht des TTA-Implantats von Figur 3;
- Figur 8: eine Draufsicht auf das TTA-Implantat von Figur 3;
- Figur 9: eine erste perspektivische Ansicht einer zweiten Ausführungsform des TTA-Implantats der Figuren 1 und 2;
- Figur 10: eine Darstellung ähnlich zu Figur 9, wobei ein in Figur 9 oberer Bereich des TTA-Implantats abgeschnitten ist;
- Figur 11: eine zweite perspektivische Ansicht des TTA-Implantats von Figur 9;
- Figur 12: eine erste perspektivische Ansicht einer dritten Ausführungsform des TTA-Implantat der Figuren 1 und 2;
- Figur 13: eine zweite perspektivische Ansicht des TTA-Implantats von Figur 12;
- Figur 14: eine dritte perspektivische Ansicht des TTA-Implantats von Figur 12;
- Figur 15: eine Darstellung ähnlich zu Figur 12, wobei ein in Figur 12 seitlicher Bereich des TTA-Implantats abgeschnitten ist;
- Figur 16: eine Draufsicht des TTA-Implantats von Figur 12;
- Figur 17: eine Seitenansicht des TTA-Implantats von Figur 12; und
- Figur 18: eine Frontansicht des TTA-Implantats von Figur 12.

Funktionsäquivalente Elemente und Bereiche tragen in unterschiedlichen Ausführungsformen die gleichen Bezugszeichen.

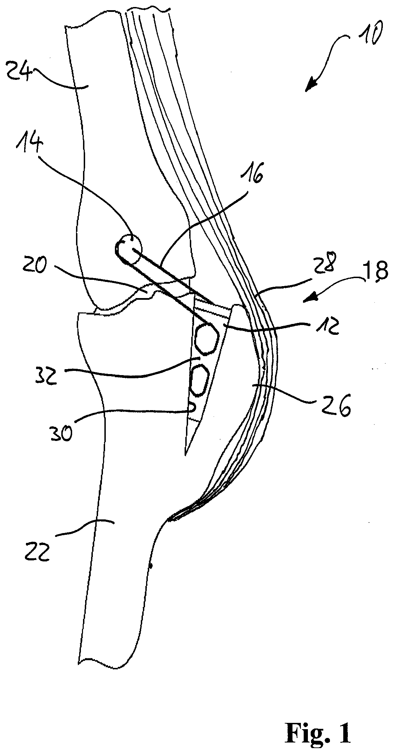

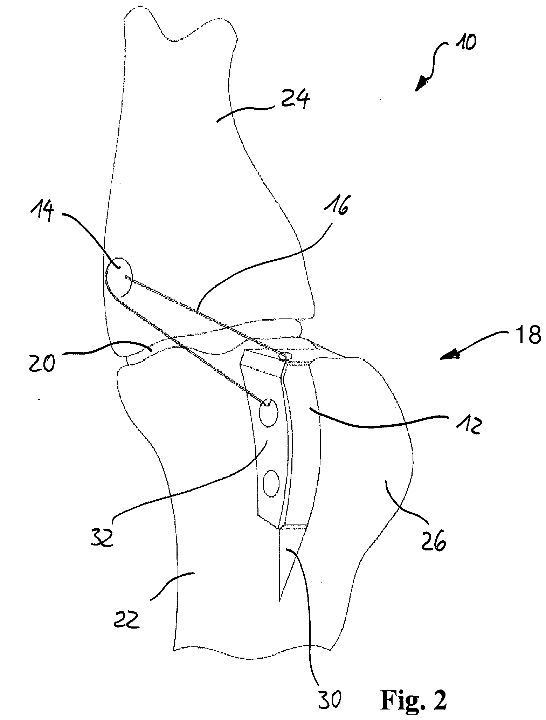

Ein TTA-Implantatsystem trägt in den Figuren 1 und 2 insgesamt das Bezugszeichen 10. Es umfasst ein TTA-Implantat 12, eine Verankerungseinrichtung in Form einer Knochenschraube 14 und ein flexibles Befestigungsmittel 16. In den Figuren 1 und 2 ist ferner ein Bereich eines Knies insgesamt mit 18 bezeichnet. Ein Kniegelenk trägt das Bezugszeichen 20. Eine abschnittsweise gezeichnete Tibia trägt das Bezugszeichen 22, ein ebenfalls abschnittsweise gezeichneter Femur das Bezugszeichen 24.

Das TTA-Implantatsystem 10 dient zur Herstellung eines "Tibial Tuberosity Advancement", abgekürzt TTA. Hierdurch wird eine Degeneration des cranialen Kreuzbands behandelt, durch die die Stabilität eines Knies beispielsweise eines Hundes oder einer Katze verringert wird. Das TTA-Implantatsystem 10 wird also vor allem in der Veterinärmedizin dazu eingesetzt, eine Tibia Tuberosity 26 nach vorn zu verlagern, wodurch der Winkel einer Patellasehne 28 (nur in Figur 1 gezeichnet) verändert wird. Hierdurch wiederum wird die tibiafemurale Scherkraft bei Belastung neutralisiert.

Hierzu wird, wie ebenfalls aus den Figuren 1 und 2 ersichtlich ist, die Tibia 22 im knienahen Bereich 18 an der Vorderseite von oben bereichsweise aufgespaltet und abgespreizt, so dass ein keilförmiger Einschnitt bzw. Knochenspalt 30 entsteht. In diesen Einschnitt 30 wird das TTA-Implantat 12 von der Seite mit einem distalen Bereich 32 voraus eingeschoben, so dass der aufgespaltene Bereich abgespreizt und die Tibia Tuberosity 26 nach vorn verlagert bleibt (der distale Bereich 32 des TTA-Implantats 12 ist also in Figur 1 dem Betrachter zugewandt).

Wie aus den Figuren 1 und 2 ersichtlich ist, ist die Verankerungseinrichtung 14 an einer vom TTA-Implantat 12 entfernten Stelle, nämlich auf der anderen Seite des Kniegelenks 20 im Femur 24 verankert. Das flexible Befestigungsmittel 16 ist einerseits an der Verankerungseinrichtung 14 und andererseits am TTA-Implantat 12 befestigt und auf diese Weise zwischen der Verankerungseinrichtung 14 und dem TTA-Implantat 12 gespannt (auf die Befestigung des flexiblen Befestigungsmittels 16 am TTA-Implantat 12 wird weiter unten noch im Detail Bezug genommen werden). Auf diese Weise wird eine Rotation von Femur 12 und Tibia 22 relativ zueinander verhindert und so das Kniegelenk 20 zusätzlich stabilisiert.

Bei dem flexiblen Befestigungsmittel 16 handelt es sich vorliegend um einen üblichen Nahtfaden, wie er in der Chirurgie Anwendung findet. Zur Erhöhung der Stabilität können auch mehrere Befestigungsmittel 16, also vorliegend mehrere Nahtfäden, zwischen der Verankerungseinrichtung 14 und dem TTA-Implantat 12 verspannt werden. Der Nahtfaden kann dabei sowohl aus einem Monofilamentmaterial oder ein aus einem Mulitfilamentmaterial hergestellt sein. In den Figuren 1 und 2 ist eine Implantatsituation gezeigt, bei der das TTA-Implantat 12 in die Tibia 22 eingesetzt ist. Es versteht sich, dass bei nicht gezeigten Ausführungsformen das TTA-Implantat auch beispielsweise in den Femur eingesetzt sein könnte.

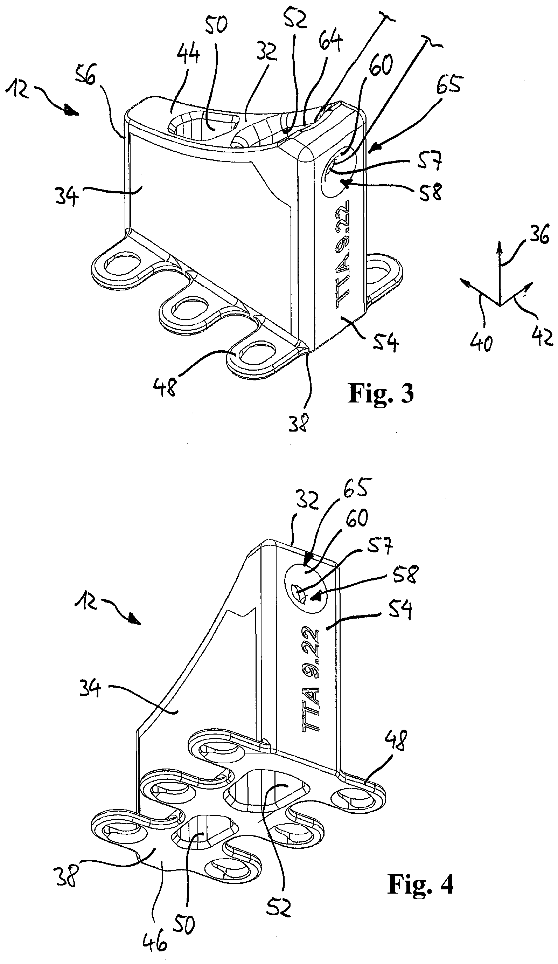

Nun wird unter Bezugnahme auf die Figuren 3-8 eine erste Ausführungsform des TTA-Implantats 12 im Detail erläutert.

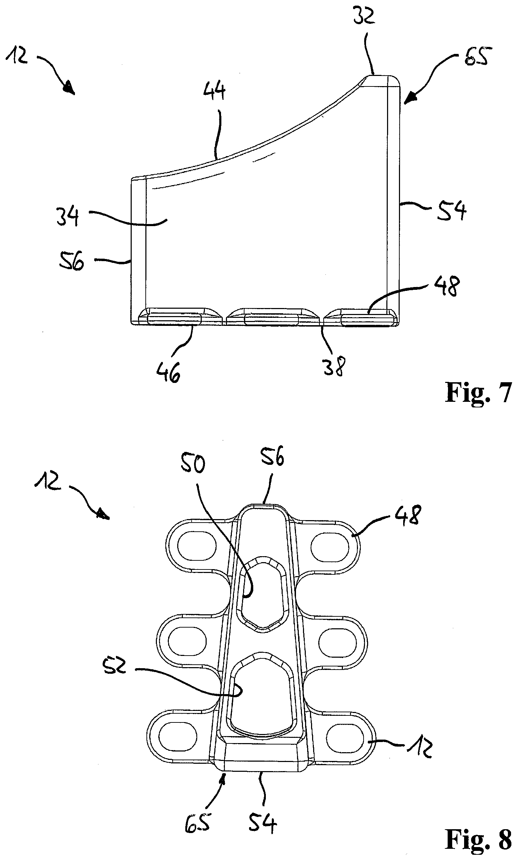

Das TTA-Implantat 12 umfasst einen vorliegend aus Metall hergestellten Implantatkörper 34. Grundsätzlich denkbar wäre aber auch, dass der Implantatkörper 34 aus Kunststoff, Kohlefaser, Polyetheretherketon, oder einem resorbierbaren Material, oder einer Kombination hiervon hergestellt ist. Er erstreckt sich längs einer ersten Richtung 36, die sich von einem proximalen Ende 38 zu dem bereits oben erwähnten distalen Ende 32 erstreckt. Der Implantatkörper 34 erstreckt sich ferner längs einer zweiten Richtung 40 und längs einer dritten Richtung 42 (das entsprechende kartesische Koordinatensystem ist allerdings nur in Figur 3 gezeichnet). Wie aus Figur 8 ersichtlich ist, verjüngt sich der Querschnitt des Implantatkörpers 34 in der zweiten Richtung 40. Wie insbesondere aus Figur 7 ersichtlich ist, verläuft ein dort oberer distaler Rand 44 von einem proximalen Rand 46 aus gesehen konvex. Der distale Rand 44 ist gegenüber dem proximalen Rand 46 insgesamt schräg.

Von jeder Seite des Implantatkörpers 34 erstrecken sich drei Befestigungslaschen 48, die insoweit eine an das proximale Ende 38 des Implantatkörpers 34 angeformte erste Befestigungseinrichtung bilden (aus Gründen der Übersichtlichkeit ist jeweils nur eine Befestigungslasche mit einem Bezugszeichen versehen). Die Befestigungslaschen 48 sind relativ zum Implantatkörper 34 biegbar, und sie dienen zur Befestigung des TTA-Implantats 12 an der Tibia 22 mittels Knochenschrauben (nicht dargestellt). Es versteht sich, dass bei anderen, nicht gezeigten Ausführungsformen auch eine andere Anzahl von Befestigungslaschen vorhanden sein kann, sowie überhaupt eine andere Art von erster Befestigungseinrichtung.

Der Implantatkörper 34 wird längs zu der ersten Richtung 36 von zwei Durchgangsöffnungen 50 und 52 durchsetzt, die sich insoweit vom proximalen Ende 38 bis zum distalen Ende 32 erstrecken. Von einer ersten Stirnseite 54, die in der ersten Richtung 36 eine größere Erstreckung aufweist als eine entgegengesetzt angeordnete zweite Stirnseite 56, erstreckt sich ein erster Abschnitt 57 eines Befestigungsmittelkanals 58. Der erste Abschnitt des Befestigungsmittelkanals 58 mündet insoweit einerseits in die erste Stirnseite 54 und andererseits in die zu der ersten Stirnseite 54 benachbarte Durchgangsöffnung 52.

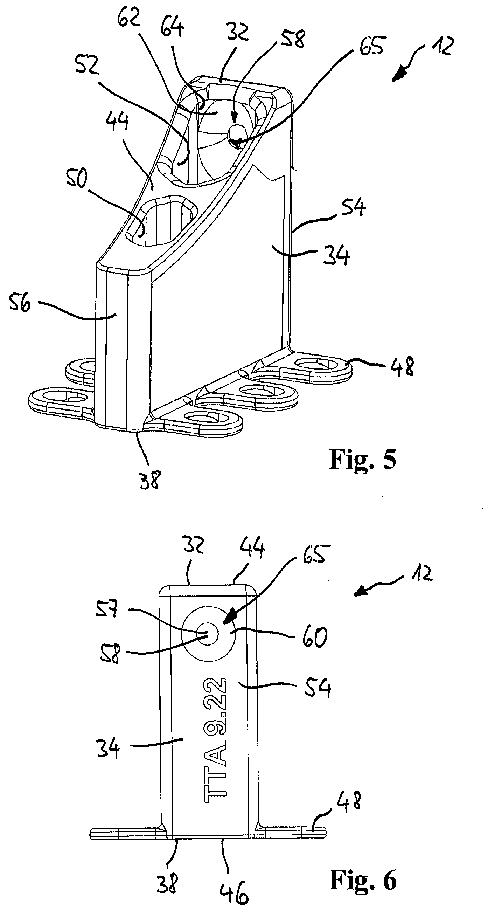

Ein Mündungsrand 60 des ersten Abschnitts 57 des Befestigungsmittelkanals 58 zur ersten Stirnseite 54 hin ist abgerundet bzw. trichterförmig ausgebildet. Der erste Abschnitt 57 des Befestigungsmittelkanals 58 hat einen kreisförmigen Querschnitt. Ein Mündungsrand 62 des ersten Abschnitts 57 zur zweiten Durchgangsöffnung 52 hin ist ebenfalls trichterförmig ausgebildet (Figur 5).

Ein Bereich der zweiten Durchgangsöffnung 52, der sich von der Mündung des ersten Abschnitts des Befestigungsmittelkanals 58 bis zum distalen Ende 32 erstreckt, bildet einen zweiten Abschnitt 64 des Befestigungsmittelkanals 58. Insoweit ist die zweite Durchgangsöffnung 52 Teil des Befestigungsmittelkanals 58. In dem Bereich des zweiten Abschnitts 64 hat der Befestigungsmittelkanal 58 einen in etwa ovalen bzw. 5-eckigen Querschnitt. Grundsätzlich könnte er auch einen elliptischen Querschnitt aufweisen.

Durch den Befestigungsmittelkanal 58 wird eine zweite Befestigungseinrichtung 65 gebildet, mittels der das flexible Befestigungsmittel 16 an dem TTA-Implantat 12 festgelegt werden kann. Dabei ist der Befestigungsmittelkanal 58 so ausgebildet und dimensioniert, dass das flexible Befestigungsmittel 16 durch ihn hindurch geführt werden kann. Dies ist schematisch in Figur 3 gezeigt.

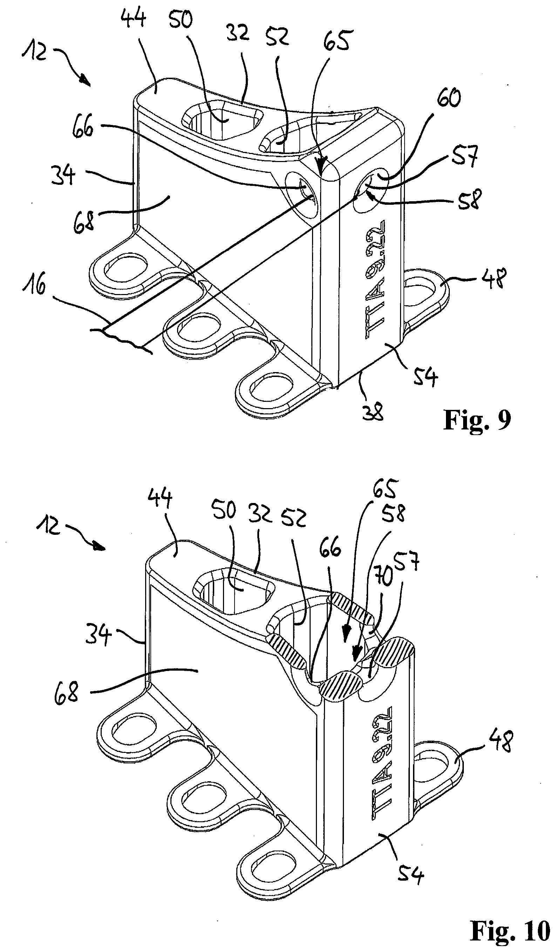

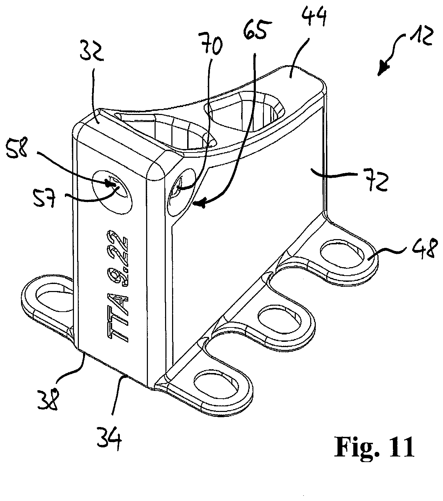

Eine zweite Ausführungsform eines TTA-Implantats 12 wird nun unter Bezugnahme auf die Figuren 9-11 erläutert. Das TTA-Implantat 12 der Figuren 9-11 ist fast identisch zu dem TTA-Implantat 12 der Figuren 3-8 ausgebildet. Allerdings umfasst der Befestigungsmittelkanal 58 einen dritten Abschnitt 66, der einerseits in die zweite Durchgangsöffnung 52 und andererseits in eine erste laterale Seitenfläche 68 (Figuren 9 und 10) mündet. Ferner umfasst der Befestigungsmittelkanal 58 auch noch einen vierten Abschnitt 70, der in eine zweite laterale Seitenfläche 72 (Figur 11) mündet, die zu der ersten lateralen Seitenfläche 68 entgegengesetzt angeordnet ist. Das flexible Befestigungsmittel 16 kann somit durch diesen dritten Abschnitt 66, wie in Figur 9 gezeichnet ist, oder durch den vierten Abschnitt 70 hindurchgeführt werden.

Die oben beschriebenen beiden Ausführungsformen eines TTA-Implantats 12 besitzen jeweils zwei Durchgangsöffnungen 50 und 52. Grundsätzlich denkbar, in der Zeichnung jedoch nicht dargestellt, ist aber auch eine Ausführungsform, bei der der Implantatkörper eine andere Anzahl von solchen Durchgangsöffnungen aufweist, sowie eine Ausführungsform, bei der der Implantatkörper überhaupt keine solchen Durchgangsöffnungen aufweist. In dem letztgenannten Fall könnte ein Befestigungsmittelkanal beispielsweise durch eine Durchgangsöffnung gebildet werden, die sich wenigstens in etwa längs zu der dritten Richtung von einer der lateralen Seitenflächen bis zu der anderen der lateralen Seitenflächen erstreckt und in diese mündet, und/oder durch eine Durchgangsöffnung gebildet werden, die sich von der ersten Stirnseite längs zur zweiten Richtung oder leicht schräg zur zweiten Richtung zum distalen Rand erstreckt und in diesen mündet.

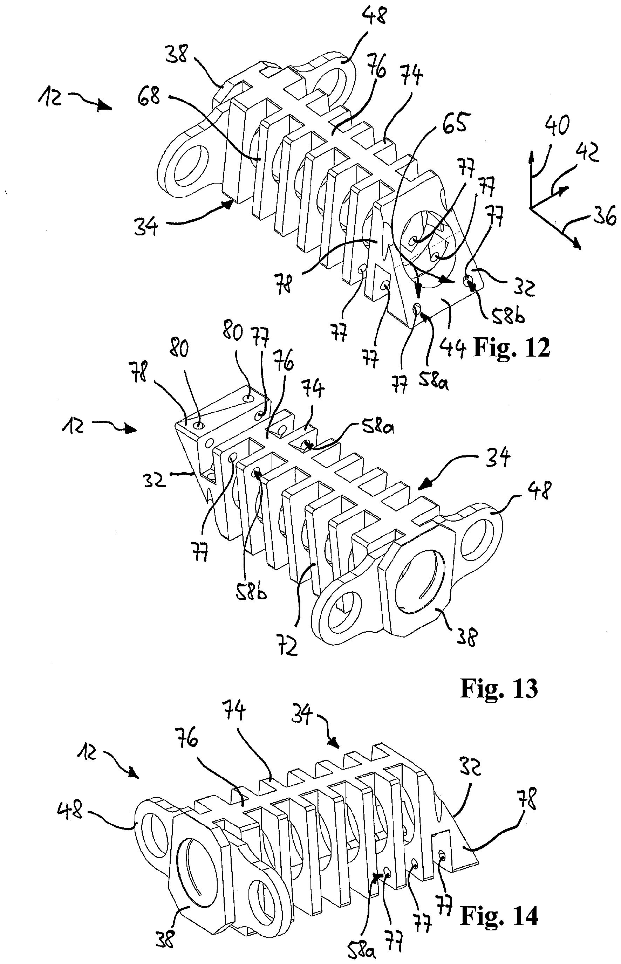

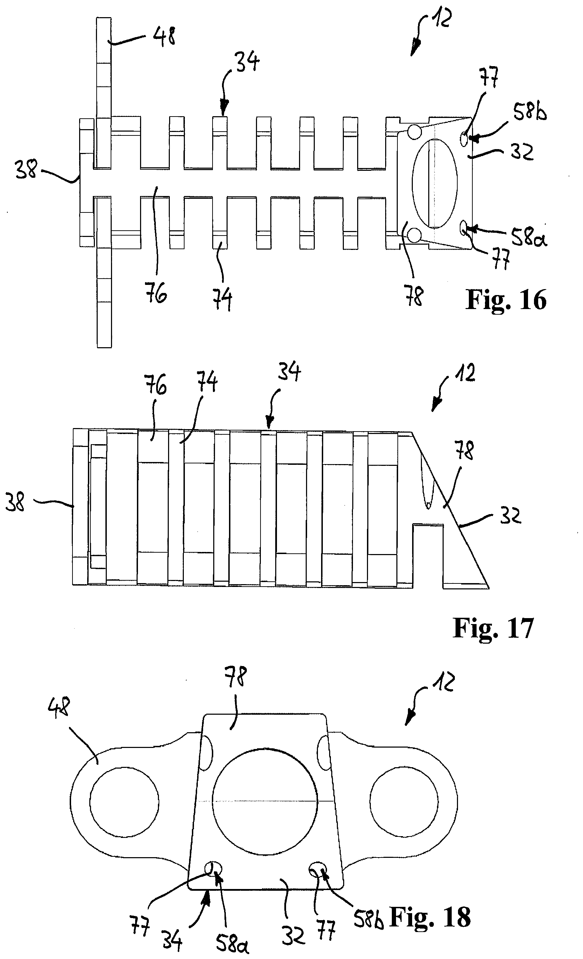

Eine dritte Ausführungsform eines TTA-Implantats 12 wird nun unter Bezugnahme auf die Figuren 12-18 erläutert. Bei dieser Ausführungsform weist der Implantatkörper 34 eine Mehrzahl von Plattenabschnitten 74 auf (aus Gründen der Übersichtlichkeit ist nur ein Plattenabschnitt mit einem Bezugszeichen versehen), die sich quer zu der ersten Richtung 36 erstrecken (man beachte, dass die Lage der Richtungen 36, 40 und 42 in Figur 12 anders ist als in Figur 3, funktional die Richtungen aber gleich sind). Die Plattenabschnitte 74 sind dabei durch einen sich längs zu der ersten Richtung 36 erstreckenden Zentralabschnitt 76 miteinander verbunden.

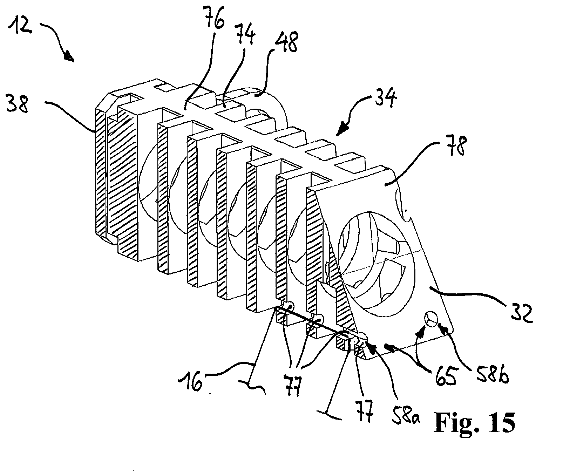

Das TTA-Implantat 12 der Figuren 12 bis 18 weist zwei Befestigungsmittelkanäle 58a und 58b auf. Diese sind durch fluchtende Durchgangsbohrungen 77 in den Plattenabschnitten 74 am distalen Ende 32 des Implantatkörpers 34 gebildet, wobei sich die Durchgangsbohrungen 77 längs der ersten Richtung 36 erstrecken. In einem End-Plattenabschnitt 78 sind ferner zwei Bohrungen 80 vorhanden (Figuren 13 und 15), die sich längs der 2. Richtung 40 von der 1. Stirnseite 54 bis in die entsprechende in der 1. Richtung 36 verlaufende Durchgangsbohrung 77 erstrecken. In Figur 15 ist beispielhaft und schematisch die Durchführung eines flexiblen Befestigungsmittels 16 durch den Befestigungsmittelkanal 58 gezeigt.

Eine weitere, nicht gezeigte Ausführungsform ist weitgehend identisch zu der letztgenannten Ausführungsform. Jedoch erstreckt sich ein Befestigungsmittelkanal beispielsweise in Form einer Durchgangsöffnung durch den End-Plattenabschnitt längs zu der dritten Richtung.

## Patentansprüche

1. TTA-Implantatsystem (10) umfassend
ein TTA-Implantat (12) umfassend
einen Implantatkörper (34) zum Einsetzen in einen Knochenspalt (30),
eine erste an einen proximalen Bereich (38) des Implantatkörpers (34) angeformte Befestigungseinrichtung (48) zum Befestigen des Implantatkörpers (12) am Knochen (22),
**dadurch gekennzeichnet, dass** das TTA-Implantat ferner umfasst:
in oder an einem distalen Bereich (32) des Implantatkörpers eine zweite Befestigungseinrichtung (65) für ein flexibles Befestigungsmittel (16); und das TTA-Implantatsystem weiterhin umfasst das flexible Befestigungsmittel (16), welches an der zweiten Befestigungseinrichtung (65) des TTA-Implantats (12) befestigt werden kann; und
eine Verankerungseinrichtung (14), die an einer vom TTA-Implantat (12) entfernten Stelle im Knochen (24) verankert werden kann und die so ausgebildet ist, dass an ihr das flexible Befestigungsmittel (16) festgelegt werden kann.

2. TTA-Implantatsystem (10) nach Anspruch 1, **dadurch gekennzeichnet, dass** die zweite Befestigungseinrichtung (65) einen Befestigungsmittelkanal (58) umfasst, der so ausgebildet und dimensioniert ist, dass durch ihn das flexible Befestigungsmittel (16) hindurchgeführt werden kann.

3. TTA-Implantatsystem (10) nach Anspruch 2, **dadurch gekennzeichnet, dass** es eine Mehrzahl von flexiblen Befestigungsmitteln (16) umfasst, die durch den Befestigungsmittelkanal (58) hindurchgeführt werden können.

4. TTA-Implantatsystem (10) nach einem der vorhergehenden Ansprüche, **dadurch gekennzeichnet, dass** das flexible Befestigungsmittel (16) einen Nahtfaden, insbesondere aus einem Monofilamentmaterial oder aus einem Mulitfilamentmaterial, umfasst.

5. TTA-Implantatsystem (10) nach einem der Ansprüche 2-4, **dadurch gekennzeichnet, dass** der Befestigungsmittelkanal (58) in eine Stirnseite des Implantatkörpers (34) mündet.

6. TTA-Implantatsystem (10) nach einem der Ansprüche 2-5, **dadurch gekennzeichnet, dass** der Befestigungsmittelkanal (58) in mindestens eine laterale Seitenfläche (68), vorzugsweise in beide lateralen Seitenflächen (68, 72) des Implantatkörpers (34) mündet.

7. TTA-Implantatsystem (10) nach einem der Ansprüche 2-6, **dadurch gekennzeichnet, dass** der Implantatkörper (34) mindestens eine Durchgangsöffnung (52) aufweist, welche Teil des Befestigungsmittelkanals (58) ist.

8. TTA-Implantatsystem (10) nach einem der vorhergehenden Ansprüche, **dadurch gekennzeichnet, dass** der Implantatkörper (34) eine Mehrzahl von Plattenabschnitten (74) aufweist, die sich quer zu einer ersten Richtung (36) des Implantatkörpers (34) erstrecken, die sich von einem proximalen Ende (38) zu einem distalen Ende (32) hin erstreckt, und dass die zweite Befestigungseinrichtung (65), insbesondere der Befestigungsmittelkanal (58a, 58b), in oder an mindestens einem der Plattenabschnitte (74) ausgebildet ist.

9. TTA-Implantatsystem (10) nach Anspruch 8, **dadurch gekennzeichnet, dass** in der ersten Richtung (36) des Implantatkörpers (34) gesehen auf beiden Seiten eines Plattenabschnitts (74) jeweils ein Befestigungsmittelkanal (58a, 58b) vorhanden ist.

10. TTA-Implantatsystem (10) nach einem der Ansprüche 2-9, **dadurch gekennzeichnet, dass** die zweite Befestigungseinrichtung (65) eine Mehrzahl von Befestigungsmittelkanälen (58) umfasst.

11. TTA-Implantatsystem (10) nach einem der Ansprüche 2-10, **dadurch gekennzeichnet, dass** ein Mündungsrand (60, 62) des Befestigungsmittelkanals (58) abgeschrägt oder abgerundet ist.

12. TTA-Implantatsystem (10) nach einem der Ansprüche 2-11, **dadurch gekennzeichnet, dass** der Befestigungsmittelkanal (58) mindestens bereichsweise einen kreisförmigen, ovalen, elliptischen oder mehreckigen Querschnitt aufweist.

13. TTA-Implantatsystem (10) nach einem der vorhergehenden Ansprüche, **dadurch gekennzeichnet, dass** sich der Querschnitt des Implantatkörpers (34) in einer zweiten Richtung (40), die sich quer zu einer ersten Richtung (36) des Implantatkörpers (34) erstreckt, die sich vom proximalen Ende (38) zum distalen Ende (32) hin erstreckt, verjüngt.

14. TTA-Implantatsystem (10) nach einem der vorhergehenden Ansprüche, **dadurch gekennzeichnet, dass** ein Rand (44) am distalen Ende (32) gegenüber einem Rand (46) am proximalen Ende (38) schräg ist.

15. TTA-Implantatsystem (10) nach einem der vorhergehenden Ansprüche, **dadurch gekennzeichnet, dass** der Implantatkörper (34) aus Metall, Kunststoff, Kohlefaser, Polyetheretherketon, einem resorbierbaren Material, oder einer Kombination hiervon hergestellt ist.

## Claims

1. TTA implant system (10) comprising
a TTA implant (12) comprising
an implant body (34) for insertion into a bone gap (30),
a first fastening device (48) formed on a proximal region (38) of the implant body (34) for fastening the implant body (34) to the bone (22),
**characterized in that** the TTA implant further comprises:
a second fastening device (65) in or on a distal region (32) of the implant body for a flexible fastening means (16); and the TTA implant system further comprises the flexible fastening means (16), which can be fastened to the second fastening device (65) of the TTA implant (12); and
an anchoring device (14) which can be anchored in the bone (24) at a point remote from the TTA implant (12) and which is designed such that the flexible fastening means (16) can be secured thereto.

2. TTA implant system (10) according to claim 1, **characterized in that** the second fastening device (65) comprises a fastening means channel (58) which is designed and dimensioned such that the flexible fastening means (16) can be guided therethrough.

3. TTA implant system (10) according to claim 2, **characterized in that** it comprises a plurality of flexible fastening means (16) which can be guided through the fixing means channel (58).

4. TTA implant system (10) according to any of the preceding claims, **characterized in that** the flexible fastening means (16) comprises a seam thread, in particular consisting of a monofilament material or a mullite filament material.

5. TTA implant system (10) according to any of claims 2-4, **characterized in that** the fastening means channel (58) opens into an end face of the implant body (34).

6. TTA implant system (10) according to any of claims 2-5, **characterized in that** the fixing means channel (58) opens into at least one lateral side surface (68), preferably into both lateral side surfaces (68, 72), of the implant body (34).

7. TTA implant system (10) according to any of claims 2-6, **characterized in that** the implant body (34) has at least one through-opening (52), which is part of the fastening means channel (58).

8. TTA implant system (10) according to any of the preceding claims, **characterized in that** the implant body (34) has a plurality of plate portions (74) extending transversely to a first direction (36) of the implant body (34) that extends from a proximal end (38) toward a distal end (32), and **in that** the second fastening means (65), in particular the fastening means channel (58a, 58b), is formed in or on at least one of the plate portions (74).

9. TTA implant system (10) according to claim 8, **characterized in that,** viewed in the first direction (36) of the implant body (34), a fastening means channel (58a, 58b) is present on both sides of a plate portion (74).

10. TTA implant system (10) according to any of claims 2-9,
**characterized in that** the second fastening device (65) comprises a plurality of fastening means channels (58).

11. TTA implant system (10) according to any of claims 2-10, **characterized in that** a mouth edge (60, 62) of the fixing means channel (58) is chamfered or rounded.

12. TTA implant system (10) according to any of claims 2-11, **characterized in that** the fastening means channel (58) has, at least in regions, a circular, oval, elliptical or polygonal cross section.

13. TTA implant system (10) according to any of the preceding claims, **characterized in that** the cross section of the implant body (34) tapers in a second direction (40) extending transversely to a first direction (36) of the implant body (34) that extends from the proximal end (38) toward the distal end (32).

14. TTA implant system (10) according to any of the preceding claims, **characterized in that** an edge (44) at the distal end (32) is oblique with respect to an edge (46) at the proximal end (38).

15. TTA implant system (10) according to any of the preceding claims, **characterized in that** the implant body (34) is made of metal, plastics material, carbon fiber, polyether ether ketone, an absorbable material, or a combination thereof.

## Revendications

1. Système d'implant TTA (10) comprenant
un implant TTA (12) comprenant
un corps d'implant (34) à insérer dans une fente osseuse (30),
un premier dispositif de fixation (48) formé sur une zone proximale (38) du corps d'implant (34) pour la fixation du corps d'implant (34) sur l'os (22),
**caractérisé en ce que** l'implant TTA comprend en outre :
dans ou sur une zone distale (32) du corps d'implant un deuxième dispositif de fixation (65) pour un moyen de fixation flexible (16) ; et le système d'implant TTA comprend en outre le moyen de fixation flexible (16), lequel peut être fixé sur le deuxième dispositif de fixation (65) de l'implant TTA (12) ; et
un dispositif d'ancrage (14), qui peut être ancré dans l'os (24) à un emplacement éloigné de l'implant TTA (12) et qui est réalisé de sorte que le moyen de fixation flexible (16) peut être immobilisé sur celui-ci.

2. Système d'implant TTA (10) selon la revendication 1, **caractérisé en ce que** le deuxième dispositif de fixation (65) comprend un canal de moyen de fixation (58), qui est réalisé et dimensionné de sorte que le moyen de fixation flexible (16) peut être guidé à travers celui-ci.

3. Système d'implant TTA (10) selon la revendication 2, **caractérisé en ce qu'**il comprend une pluralité de moyens de fixation flexibles (16), qui peuvent être guidés à travers le canal de moyen de fixation (58).

4. Système d'implant TTA (10) selon l'une quelconque des revendications précédentes, **caractérisé en ce que** le moyen de fixation flexible (16) comprend un fil de suture, en particulier composé d'un matériau monofilament ou d'un matériau multifilament.

5. Système d'implant TTA (10) selon l'une quelconque des revendications 2 à 4, **caractérisé en ce que** le canal de moyen de fixation (58) débouche dans une face frontale du corps d'implant (34).

6. Système d'implant TTA (10) selon l'une quelconque des revendications 2 à 5, **caractérisé en ce que** le canal de moyen de fixation (58) débouche dans au moins une surface latérale (68), de préférence dans les deux surfaces latérales (68, 72) du corps d'implant (34).

7. Système d'implant TTA (10) selon l'une quelconque des revendications 2 à 6, **caractérisé en ce que** le corps d'implant (34) présente au moins une ouverture de passage (52), laquelle fait partie du canal de moyen de fixation (58).

8. Système d'implant TTA (10) selon l'une quelconque des revendications précédentes, **caractérisé en ce que** le corps d'implant (34) présente une pluralité de parties de plaque (74), qui s'étendent transversalement par rapport à une première direction (36) du corps d'implant (34), qui s'étend à partir d'une extrémité proximale (38) vers une extrémité distale (32), et que le deuxième dispositif de fixation (65), en particulier le canal de moyen de fixation (58a, 58b), est réalisé dans ou sur au moins une des parties de plaque (74).

9. Système d'implant TTA (10) selon la revendication 8, **caractérisé en ce que** vu dans la première direction (36) du corps d'implant (34) respectivement un canal de moyen de fixation (58a, 58b) est présent sur les deux faces d'une partie de plaque (74).

10. Système d'implant TTA (10) selon l'une quelconque des revendications 2 à 9, **caractérisé en ce que** le deuxième dispositif de fixation (65) comprend une pluralité de canaux de moyen de fixation (58).

11. Système d'implant TTA (10) selon l'une quelconque des revendications 2 à 10, **caractérisé en ce qu'**un bord d'embouchure (60, 62) du canal de moyen de fixation (58) est biseauté ou arrondi.

12. Système d'implant TTA (10) selon l'une quelconque des revendications 2 à 11, **caractérisé en ce que** le canal de moyen de fixation (58) présente au moins par zones une section transversale circulaire, ovale, elliptique ou polygonale.

13. Système d'implant TTA (10) selon l'une quelconque des revendications précédentes, **caractérisé en ce que** la section transversale du corps d'implant (34) s'amincit dans une deuxième direction (40), qui s'étend transversalement par rapport à une première direction (36) du corps d'implant (34), qui s'étend à partir de l'extrémité proximale (38) vers l'extrémité distale (32).

14. Système d'implant TTA (10) selon l'une quelconque des revendications précédentes, **caractérisé en ce qu'**un bord (44) sur l'extrémité distale (32) est incliné par rapport à un bord (46) sur l'extrémité proximale (38).

15. Système d'implant TTA (10) selon l'une quelconque des revendications précédentes, **caractérisé en ce que** le corps d'implant (34) est fabriqué à partir de métal, de matière plastique, de fibres de carbone, de polyétheréthercétone, d'un matériau résorbable, ou d'une combinaison de ceux-ci.
